# EUROPÄISCHE PATENTSCHRIFT

(11) **EP 1 458 281 B1**
(45) Veröffentlichungstag und Bekanntmachung des Hinweises auf die Patenterteilung: **16.12.2009**
(21) Anmeldenummer: 02793106.2
(22) Anmeldetag: 20.12.2002
(51) Int. Cl.: A61B 5/00

(54) **Unterscheidung von Herzrhythmen mittels Poincare- oder Lorenzfiguren.**
Distinction of cardiac rhythms by Poincare- or Lorenzfigures.
Différenciation des rythmes cardiaques par des figures Poincare ou Lorenz.

(30) Priorität: 21.12.2001 DE 10163348
(43) Veröffentlichungstag der Anmeldung: 22.09.2004
(73) Patentinhaber: Esperer, Hans, 39130 Magdeburg (DE)
(72) Erfinder: Esperer, Hans, 39130 Magdeburg (DE)
(74) Vertreter: Reitstötter - Kinzebach
(86) Internationale Anmeldenummer: PCT/EP2002/014689
(87) Internationale Veröffentlichungsnummer: WO 2003/053233

(56) Entgegenhaltungen:
- DE-A- 3 701 947
- ANDREAS GROTE, DR. WOLFGANG STIELER: "Herzschlag und Stimme" C'T, ISSN 0724-8679, Bd. 1, 2001, Seite 36 XP001146973
- HAGEN KNAF, PATRICK LANG: "Risikoabschätzung für die Erkrankung an Herzrhythmusstörungen / Plötzlichem Herztod" INTERNET ARTICLE, [Online] 17. Dezember 2001 (2001-12-17), XP002239397 Gefunden im Internet: <URL:http://www.itwm.de/as/projects/ekg/ek g_dt.html> [gefunden am 2003-04-23]
- G SCHMIDT, G E MORFILL: "Complexity diagnostics in cardiology: methods" PACING CLIN ELECTROPHYSIOL, Bd. 17, Nr. 12/1, Dezember 1994 (1994-12), Seiten 2336-41, XP009009775
- H V HUIKURI: "Heart rate dynamics and vulnerability to ventricular tachyarrhythmias" ANNALS OF MEDICINE, Bd. 29, Nr. 4, August 1997 (1997-08), Seiten 321-5, XP009009777
- M A WOO, W G STEVENSON, D K MOSER: "Comparson of four methods of assessing heart rate variability in patients with heart failure" AM J CRIT CARE, Bd. 5, Nr. 1, Januar 1996 (1996-01), Seiten 34-41, XP009009768
- H V HUIKURI, T SEPPÄNEN, ET AL: "Abnormalities in beat-to-beat dynamics of heart rate before the spontaneous onset of life-threatening ventricular tachyarrhythmias in patients with prior myocardial infarction" CIRCULATION, Bd. 93, Nr. 10, 15. Mai 1996 (1996-05-15), Seiten 1863-44, XP009009770
- F AZUAJE, W DUBITZKY, ET AL: "Predicting coronary disease risk based on short-term RR interval measurements: a neural network approach" ARTIFUCIAL INTELÖLIGENCE IN MEDICINE, Bd. 15, 1999, Seiten 275-297, XP002239396

## Beschreibung

Die vorliegende Erfindung betrifft ein Verfahren und eine Vorrichtung zur automatisierten Detektion und Differenzierung von Herzrhythmusstörungen.

Das menschliche Herz ist ein für unsere Lebensfähigkeit essentielles, hochentwickeltes und komplexes elektromechanisches System. Das Herz, das während eines 24-Stunden-Tages typischerweise zwischen 100.000 und 140.000 mal schlägt, ist zwar einerseits der willkürlichen Kontrolle des Menschen entzogen aber andererseits über die Nerven des sympathischen und parasympathischen Nervensystems mit allen für die Kreislaufregulation wichtigen Organsystemen aufs Engste verknüpft, so dass seine Auswurfleistung kontinuierlich den ständig wechselnden Bedürfnissen des Kreislaufsystems angepasst werden kann. Das Herz kann bei Bedarf sowohl seine Schlagkraft als auch seine Schlagfrequenz innerhalb weniger Millisekunden erhöhen oder herabsetzen. Aufgrund der komplexen zentralnervösen, respiratorischen, zirkulatorischen und thermoregulatorischen Einflüsse stellt der Herzschlag keine konstante Größe dar, sondern zeigt - auch beim gesunden Menschen - eine unregelmäßige, geradezu "chaotische" Rhythmik, die als Herzfrequenzvariabilität (HRV) bezeichnet wird.

Neuere wissenschaftliche Untersuchungen haben gezeigt, dass das kardiale autonome Nervensystem in einem direkten Zusammenhang mit der Entstehung von Herzrhythmusstörungen aus dem Bereich der Vorhöfe (supraventrikuläre Arrhythmien) und der Kammern (ventrikuläre Arrhythmien) steht. Daher haben Untersuchungen der Herzfrequenzvariabilität eine zunehmende Bedeutung in der Kardiologie gewonnen. Bislang beschränken sich Untersuchungen der Herzfrequenzvariabilität allerdings auf die Erfassung des sog. "autonomen Status" oder auf die Einschätzung des kardialen Arrhythmierisikos. In der klinischen Diagnostik und Differentialdiagnostik werden Untersuchungen der Herzfrequenzvariabilität bislang aber nicht eingesetzt.

Vor allem im Zusammenhang mit dem sog. Eventmonitoring und dem telemedizinischen Rhythmusmonitoring, das auch Homemonitoring bezeichnet wird, hat in den letzten Jahren außerdem die kontinuierliche elektrokardiographische Überwachung von Patienten mit hohem Risiko für spontane supraventrikuläre und ventrikuläre Arrhythmien zunehmend an Bedeutung gewonnen.

Zur Arrhythmieüberwachung werden derzeit vor allem nichtinvasive, bei bestimmten Indikationen, beispielsweise bei unklarer Synkope, auch invasive Techniken eingesetzt.

Die bislang verfügbaren nichtinvasiven elektrokardiographischen (EKG) Überwachungsmethoden bestehen bei ambulanten Patienten aus dem klassischen 24-Stunden-Holter-Monitoring, sowie dem Eventmonitoring und der telemetrischen Überwachung. Die modernen Eventrecorder erlauben eine Fernübertragung kurzer Segmente eines Elektrokardiogramms (EKGs) von 30 Sekunden bis 5 Minuten Dauer mittels Telefonmodem oder über Mobiltelefon, wobei moderne Eventrecorder bereits in die Mobiltelefone integriert sein können.

Die Arrhythmiedetektion in den Holter-Analysegeräten erfolgt nach unterschiedlichen Verfahren, wobei vor allem das sogenannte "Template Matching" und die "Feature Extraction" (Kennedy et al., Am J Noninvasive Cardiol 1992; 6: 137-146), sowie die QRS-Flächenmethode (Neilson et al., Computers in Cardiology, IEEE catalogue 1974; 74: 379) weit verbreitet sind. Neuerdings werden auch neuronale Netzwerke zur Auswertung von EKG-Daten herangezogen. Nicht lineare dynamische Verfahren wurden zwar bereits von Igel et al. in J Cardiovasc Electrophysiol 1997; 8: 388-397 theoretisch diskutiert, sind aber bislang noch in kein im klinischen Alltag verwendbares Analysesystem implementiert worden. In dem Patent US 6,192,273 (Igel et. al.) wird ein automatisches Verfahren zur Klassifikation des Herzrhythmus beschreiben, welches eine Detektion von normalen Rhythmus, monomorpher Tachykardie und polymorpher Tachykardie ermöglichen soll. Dabei werden die Zykluslänge und die Regularität von 5 Sekunden langen Messintervallen ausgewertet.

Bei stationären Patienten erfolgt die Rhythmusüberwachung entweder mittels bettseitigem EKG-Monitor oder zentral über eine EKG-Überwachungseinheit auf der Intensivstation.

Als invasive Methode sind derzeit implantierbare Rhythmusmonitore der Firmen Reveal und Medtronic auf dem Markt, die für den Nachweis von Bradyarrhythmien gedacht sind. Dabei wird das EKG-Signal gespeichert und muss von einem externen Telemetriegerät ausgelesen und anschließend von einem Experten beurteilt werden. Eine automatische Arrhythmiedetektion ist nicht möglich.

Die bisher bekannten Arrhythmieüberwachungs- und Detektionsverfahren sind mit zahlreichen Nachteilen behaftet.

So ist ein wesentlicher Nachteil der bekannten Langzeit-EKG-Analysesysteme darin zu sehen, dass die Analysesysteme von einem auswertenden Experten zunächst bezüglich des Grundrhythmus angelernt werden müssen. Darüber hinaus muss üblicherweise eine vom untersuchenden Arzt geführte Validierung der vom Analysesystem zunächst erkannten Arrhythmieformen durchgeführt werden. Schließlich ist das Spektrum der detektierbaren und differenzierbaren Arrhythmieformen angesichts der großen Vielfalt klinisch relevanter Herzrhythmusstörungen sehr klein und umfasst typischerweise die folgenden Rhythmus- bzw. Arrhythmie-Typen:
- Sinusrhythmus
- Supraventrikuläre Extrasystole (SVES)
- Ventrikuläre Extrasystole (VES) bzw. aberrante QRS-Komplexe
- Supraventrikuläre Tachykardien (SVT)
- Ventrikuläre Tachykardien (VT)
- Pausen
- WI-Schrittmacheraktivität (also Aktivität eines permanent implantierten Einkammer-Bedarfsschrittmachers)
- DDD-Schrittmacheraktivität (Aktivität eines Zweikammerschrittmachers, der in verschieden Modi (wie WD, VAT oder DDD) arbeiten kann).

(Die Abkürzungen WI, WD, VAT und DDD entsprechen einem international anerkannten Schrittmacher-Kode).

Während durch technologische Neuerungen und eine damit verbundene Verbesserung der Signalqualität die diagnostische Effizienz bei der Detektion ventrikulärer Arrhythmien beträchtlich erhöht werden konnte, stellt die Erkennung und Differenzierung von supraventrikulären Arrhythmien noch immer ein ungelöstes Problem dar. Wegen der Kleinheit der an der Körperoberfläche abzugreifenden Potentiale, welche die atriale elektrische Aktivität repräsentieren, und wegen dem hohen, durch Muskelpotentiale und Bewegungsartefakte hervorgerufenen Rauschpegel, entziehen sich die atrialen und supraventriukulären Arrhythmien bislang völlig der Detektion und Differenzierung. Zwar gelingt es bei Vorliegen eines normalen Herzrhythmus (d.h. eines Sinusrhythmus) durch komplizierte Verstärkungs- und Mittelungsverfahren, die P-Wellen zu erfassen und zu vermessen. Diese Verfahren sind aber nur in Ruhe möglich und scheitern sobald der Patient normaler Aktivität nachgeht, so dass sie sich beispielsweise nicht für ambulante Überwachungsverfahren eignen. Außerdem wird mit den bislang bekannten Verfahren stets ein gemittelter Sinusrhythmus analysiert, so dass keine Schlag-zu-Schlag-Diagnostik möglich ist. Darüber hinaus eignen sich diese Mittelungsverfahren nur, wenn P-Wellen vorhanden sind, d.h. sie können nicht für die Diagnostik von atrialen Arrhythmien herangezogen werden können, die durch das Fehlen von P-Wellen gekennzeichnet sind und die Mehrzahl der atrialen Arrhythmien ausmachen.

Ebenso ermöglichen die bislang für das Eventmonitoring eingesetzten Systeme keine automatische Arrhythmiedetektion, geschweige denn eine Arrhythmie-Differenzierung, da nach wie vor die Arrhythmieanalyse durch einen Experten überprüft werden muss.

Auch die im Klinikbetrieb eingesetzten telemetrischen Systeme, welche die Erkennung ventrikulärer Arrhythmien (beispielsweise isolierte VES, repetitive VES oder Kammertachykardien) und Pausen ermöglichen, sind für eine Detektion und Differenzierung von supraventrikulären Arrhythmien praktisch nicht geeignet. Zudem handelt es sich bei diesen Systemen um stationäre Auswertegeräte, zu denen das EKG-Signal mittels tragbarer EKG-Transmitter übertragen werden muss. Aufgrund der begrenzten Leistung der Transmitter müssen sich die Patienten stets in enger Nachbarschaft zur Zentralstation aufhalten, so dass eine ambulante Rhythmusüberwachung unter realistischen Bedingungen des täglichen Lebens nicht möglich ist.

Eine realistischere Patientenüberwachung wird durch eine EKG-Telemetrie über Mobiltelefone ermöglicht. Allerdings begrenzt die geringe Bandbreite der Mobiltetefonsysteme die Länge der EKG-Abschnitte, die auf diese Weise übertragen werden können, drastisch.

Der vorliegenden Erfindung liegt daher das technische Problem zugrunde, eine Vorrichtung zur Detektion und Differenzierung von Herzrhythmusstörungen bereitzustellen, welche eine vollautomatische Erkennung von klinisch relevanten Arrhythmien ermöglicht, die nicht mehr von Experten validiert werden muss. Die Vorrichtung soll sowohl bettseitig direkt am Patienten, als auch über eine zentrale Überwachungseinheit, beispielsweise eine Telemetriestation, oder auch im telematischen Betrieb für die Patientenfernüberwachung, beispielsweise beim sogenannten Homemonitoring, und auch in implantierbaren Geräten, wie Schrittmachern und Defibrillatoren eingesetzt werden können.

Gelöst wird dieses technische Problem durch die Vorrichtung zur automatisierten Detektion und Differenzierung von Herzrhythmusstörungen gemäß vorliegendem Anspruch 1. Vorteilhafte Weiterbildungen der erfindungsgemäßen Vorrichtung sind Gegenstände der abhängigen Ansprüche.

Erfindungsgemäß werden bei der Vorrichtung zur automatisierten Detektion und Differenzierung von Herzrhythmusstörungen Messwerte einer zusammenhängenden Zeitreihe eines Herzfrequenzsignals miteinander korreliert und die korrelierten Daten in einen mehrdimensionalen Phasenraum transformiert, die in den Phasenraum transformierten Daten morphologisch charakterisiert und bestimmten vorgegebenen Mustern zuordnet und schließlich aus den zugeordneten Mustern aufgrund von empirisch gefundenen Relationen bestimmte klinisch relevante Rhythmus- bzw. Arrhythmieformen ermittelt.

Die vorliegende Erfindung schlägt demnach eine nichtlineare Analyse der Herzfrequenzvariabilität vor, wobei die ursprünglichen Messwerte einer Zeitreihe, nämlich der aufeinanderfolgenden Herzzyklen, in einem Phasenraum dargestellt werden. Der Erfindung liegt die Erkenntnis zugrunde, dass in der Phasenraumdarstellung Muster identifiziert werden können, die mit hoher Sensitivität und Spezifität bestimmten klinisch relevanten Rhythmus- bzw. Arrhythmieformen zugeordnet werden können. Die Zuordnung erfolgt dabei aufgrund von empirisch gefundenen Relationen, d.h. aufgrund von Daten aus klinischen Studien, bei denen Phasenraummuster von Patienten, deren Rhythmus- bzw. Arrhythmieformen bekannt sind, ermittelt und klassifiziert wurden.

Die Erfindung erlaubt die vollautomatische Detektion und Differenzierung eines großen Spektrums von klinisch relevanten supraventrikulären und ventrikulären Tachy- und Bradyarrhythmien.

Die Korrelation der Messwerte der Zeitreihe und die Transformation der korrelierten Daten in einen mehrdimensionalen Phasenraum erfolgt bevorzugt mittels nichtlinearer Verfahren, beispielsweise einem Schema, das in der mathematischen Terminologie als "Return Map" bezeichnet und bislang insbesondere zur physikalischen Analyse von dynamischen Prozessen verwendet wird. Besonders bevorzugt korreliert man die Messwerte der zusammenhängenden Zeitreihe des Herzfrequenzsignals über eine sog. Poincaré-Abbildung und stellt die korrelierten Daten in einem sog. Lorenzplot dar. Ein Lorenzplot, der in der Literatur auch als Poincaréplot oder Streudiagramm (engl.: "scattergram") bezeichnet wird, ist eine graphische Abbildung, bei der die Intervalllänge eines Herzzyklusses gegen ein (oder mehrere) vorhergehende Herzzyklusintervalle aufgetragen wird. Von einem allgemeinen zweidimensionalen (2D) Lorenzplot spricht man, wenn die Intervalllänge *Tⁿ* des n-ten Herzzyklusses gegen die Intervalllänge *T^{n-m}* aufgetragen wird, wobei m eine konstante ganzzahlige Verzögerung ist. Im Fall von *m*=1, d.h. der Abbildung eines Herzzyklusintervalls gegen das unmittelbar vorhergehende Intervall, spricht man von einem regulären 2D-Lorenzplot. Die resultierende lokale Punktdichte eines zweidimensionalen Lorenzplots kann graphisch beispielsweise durch unterschiedliche Grauabstufungen oder eine definierte farbliche Darstellung zum Ausdruck gebracht werden. Es ist auch möglich, die Datenpunkte des 2D-Lorenzplots in Form eines sogenannten 3D-Lorenzdiagramms darzustellen, wobei über der (*Tⁿ*, *T^{m-m}*)-Ebene die Zahl der Punkte i aufgetragen wird, die sich innerhalb einer Flächeneinheit mit Kantenlänge τ des 2D-Lorenzplots befinden. Das 3D-Diagramm erlaubt daher eine bessere Veranschaulichung der Punktdichte des 2D-Lorenzplots.

In der Literatur sind darüber hinaus dreidimensionale Lorenzplots bekannt, bei denen der Phasenraum beispielsweise durch eine Poincaré-Abbildung von drei Herzfrequenzintervallen (*Tⁿ*, *Tⁿ⁻¹*, *Tⁿ⁻²*) aufgespannt wird.

In einer grundlegenden Arbeit von Woo et al., Am Heart J 1992; 123: 704-710*,* wurde das zeitliche Verhalten der RR-Intervalle aus einem 24h-Holter-EKGs mittels Lorenzplots visualisiert. Es konnten lediglich gesunde von herzkranken Probanten unterschieden werden, wobei Woo aus dem Muster der Herzfrequenzvariabilität auf den Schweregrad der Herzinsuffizienz schließen konnte. Arrhythmien können mit der Methode von Woo et al. aber nicht festgestellt werden. Anwendungen der Lorenzplots zur Untersuchung der Herzfrequenzvariabilität wurden auch von Kamen und Tonkin in Aus NZ J Med 1995; 25: 18-16 beschrieben. Eine Möglichkeit, Lorenzplot zur automatischen Detektion und Differenzierung von Arrhythmien heranzuziehen, wird bei Kamen und Tonkin allerdings nicht erwähnt. Aus einer neueren Arbeit von Azuaje et al., Artificial Intelligence in Medicine 1999; 15: 275-297, werden die Grenzen die Methodik selbst für eine Bestimmung der Herzfrequenzvariabilität deutlich. Eine Detektion von Arrhythmien wurde auch in dieser neuen Arbeit nicht in Betracht gezogen. In einer Arbeit von Huikuri et al., Circulation 1996;93:1836-1844, American Heart Association wird die Herzfrequenzvariabilität von Patienten mit bekannter Arrhythmie mittels Lorenzplots visualisiert. Dabei wurde speziell die Frage untersucht, ob der Sympatikus oder der Vagus ursächlich für das Auftreten von Tachyarrhythmien ist. Auch diese Untersuchen zeigen keine Möglichkeit zur Diagnostik von Arrhythmien auf.

Demgegenüber liegt dem erfindungsgemäßen Verfahren die Beobachtung zugrunde, dass ein hochaufgelöster Lorenzplot eine Vielzahl von Informationen enthält, die sich als geometrisch und topologisch quantitativ beschreibbare Muster charakterisieren lassen, wobei die Muster über vorausgehende empirische Untersuchungen bestimmten klinisch relevanten Rhythmus- bzw. Arrhythmieformen zugeordnet werden können. Als besonders vorteilhaft hat sich dabei die Darstellung als 2D-Lorenzplot erwiesen, wobei vorteilhaft aber auch Häufigkeitsverteilungen im Sinne eines 3D-Lorenzdiagramms berücksichtigt werden.

Die automatische, rechnergestützte morphologische Charakterisierung eines Lorenzplots kann mittels verschiedenster Verfahren durchgeführt werden, wie sie aus dem Bereich der Mustererkennung bekannt sind. Beispielsweise können neuronale Netzwerke trainiert werden, um die bei einem Patienten ermittelten Lorenzplots mit bestimmten vorgegebenen, in einer Datenbank gespeicherten Lorenzplots zu korrelieren, bei denen die Zuordnung zu bestimmten Rhythmus- bzw. Arrhythmieformen bekannt ist. Je nach Datenmenge eines 2D-Lorenzplots oder eines 3D-Lorenzdiagramms können die Daten vor der Musterzuordnung auch nachbearbeitet, beispielsweise durch Mittelung in einem bestimmten Flächenbereicht geglättet werden.

Als besonders bevorzugt haben sich zur morphologischen Charakterisierung der Lorenzplots aber die sogenannte Clusteranalyseverfahren erwiesen. Dabei wird der Lorenzplot in an sich bekannter Weise ausgehend von vorgegebenen Startpositionen systematisch abgerastert und alle in einem bestimmten Radius der Startpositionen liegenden Punkte zu einem Minicluster zusammengefasst. Außerdem wird die mittlere Anzahl an Punktnachbarn berechnet. Unterschreitet diese einen bestimmten Grenzwert, der als Nachbarschaftsschwelle bezeichnet wird, so wird ein Clusterrand definiert, andernfalls wird weitergesucht, bis die Nachbarschaftsschwelle erreicht ist. Auf diese Weise entstehen größere Cluster, die allseits begrenzt sind. Die Abstandsverteilung der so detektierten Cluster wird bestimmt und bei Unterschreiten eines bestimmten Grenzwertes werden zusammenhängende Cluster zu einem Großcluster fusioniert. Dieser Grenzwert, der letztlich das Abbruchkriterium für die Clusterbildung darstellt, wird anhand empirischer Erfahrungen so bestimmt, dass die resultierenden Clustermuster soweit vereinfacht sind, dass eine sinnvolle Korrelation mit empirisch gesicherten Rhythmus- bzw. Arrhythmieformen gewährleistet ist. Wählt man den Grenzwert zu gross, zeigt jeder Lorenzplot entsteht letztlich einen einzigen, weitgehend undifferenzierten Großcluster, der keine diagnostischen Unterscheidungen mehr zulässt. Wählt man dagegen den Grenzwert zu klein, entsteht eine große Zahl unterschiedlicher Muster, deren morphologischen Unterschiede keinen entsprechend differenzierbaren Arrhythmieformen mehr entsprechen. Ein geeignetes Verfahren zur Clusterbildung ist beispielsweise in Lanzarini et. al., First Int'I Workshop on Image and Signal Processing and Analysis,14.-15.06.2000, Pula, Kroatien, Seiten 75-80 beschrieben.

Die ermittelten Muster werden geometrisch bezüglich Rand- und Mittelpunktskoordinaten parametrisiert, beispielsweise in Form einer Vektormatrix mit Polarkoordinaten. Die Ortsvektoren des Clusterrands können bezüglich des Koordinatenursprungs in distale und proximale Ortsvektoren unterteilt werden. Außerdem werden die lokalen Maxima der Einzelcluster analysiert. Die so ermittelten geometrischen Formkriterien und Häufigkeitsmaße werden über eine Entscheidungsmatrix bestimmtem Musterklassen zugeordnet. Die Zuordnung erfolgt vorzugsweise wiederum unter Verwendung von neuronalen Netzwerken, unter Verwendung von Methoden der Fuzzy-Logik, wie beispielsweise Fuzzy-Clustering und/oder Fuzzy-Control, oder, besonders bevorzugt, unter Verwendung von Methoden der künstlichen Intelligenz, wie beispielsweise des fallbasierten Schließen (Case Based Reasoning; CBR-Methode). Die vorgegebenen Muster sind nun wiederum in einer Datenbank mit bestimmten klinisch relevanten Rhythmus- bzw. Arrhythmieformen korreliert, so dass nach Zuordnung eines gemessenen Musters mit einem vorgegebenen Muster mit Hilfe der Datenbank die entsprechende Detektion und Differenzierung von Herzrhythmusstörungen ermöglicht wird.

Die Messwerte der zusammenhängenden Zeitreihe können über beliebig lange Zeiträume gesammelt und ausgewertet werden. Beispielsweise ist es denkbar, die Messwerte einer 24-Stunden-Messung in einem einzigen Lorenzplot darzustellen. Allerdings liefert eine solche Darstellung eine Überfülle an Information, und es besteht insbesondere die Gefahr, dass einzelne, klinisch relevante Informationen nicht mehr sinnvoll detektiert werden können. Die anfallenden Daten werden in kürzere Zeitreihen segmentiert, wobei die Messwerte der zusammenhängenden Zeitreihe des Herzfrequenzsignals einen Zeitraum von 1 bis 15 Minuten, vorzugsweise von 1 bis 10 Minuten und besonders bevorzugt etwa einen Zeitraum von 5 Minuten umfassen. Eine erste sinnvolle Zuordnung einer Herzfrequenzmessung zu einem bestimmten Muster kann typischerweise nach ca. 30 Herzzyklen, also nach einer Messdauer von ca. 1 Minute erfolgen. In bestimmten Fällen, beispielsweise bei einer kontinuierlichen Patientenüberwachung, können bestimmte klinisch relevante Frühindikatoren für Arrhythmien jedoch auch mit wesentlich kürzerer Zeitauflösung registriert werden. So ist bekannt, dass sich bestimmte lebensbedrohliche Arrhythmien, beispielsweise Tachykardien, häufig durch eine Reduktion der Herzfrequenzvariabilität (eine sogenannten "Frequenzstarre") ankündigen. Ein solcher Frühindikator einer bevorstehenden Arrhythmie kann in der laufenden Überwachung oft auch schon nach weniger als 30 Herzzyklen erkannt werden kann.

Gemäß einer besonders bevorzugten Ausführungsform der Erfindung enthält die Datenbank mit den empirisch gefundenen Relationen zwischen Mustern und Rhythmus- bzw. Arrhythmieformen sowohl eine Langzeitdatenbasis (beispielsweise auf der Grundlage von 24-Stunden-Daten), als auch eine Kurzzeitdatenbasis (beispielsweise auf der Grundlage von 5-Minuten-Segmenten).

Vorzugsweise werden bei der Ermittlung der klinisch relevanten Rhythmus- bzw. Arrhythmieformen nicht nur die morphologischen Daten der reine Mustererkennung, sondern auch die Quantifizierung der Herzfrequenzvariabilität berücksichtigt. Diese kann im Phasenraum oder im Zeitbereich, bevorzugt aber sowohl im Phasenraum als auch im Zeitbereich erfolgen.

Als Herzfrequenzsignal können unterschiedlichste Messgrößen verwendet werden, die eine kontinuierliche Überwachung der Herzfrequenz erlauben, beispielsweise die Pulskurve oder die Blutdruckkurve. Besonders bevorzugt wird allerdings ein digitalisiertes EKG-Signal verwendet, das sich im Zeitbereich mit an sich bekannten Verfahren auswerten lässt. Dabei kann das EKG als analoges Signal direkt vom Patienten, beispielsweise über die korrigierten orthogonalen Ableitungen x, y und z nach Frank aufgezeichnet werden. Die EKG-Ableitungen werden, vorzugsweise nach Bandpassfilterung und Verstärkung, analog-digital konvertiert. Die Auflösung liegt vorteilhaft im Bereich von 8 bis 24 bit und die Abtastrate beträgt vorteilhaft 100 Hz bis 1 kHz.

Als Eingangssignal können auch bereits digitalisierte EKG-Signale verwendet werden, die telemetrisch, beispielsweise über Internet oder eine digitale Telefon- oder Datenleitung an eine Analyseeinrichtung übermittelt werden. Prinzipiell kann die Datenauswertung auch auf der Grundlage eines Einkanal-EKGs erfolgen.

Zur Bestimmung der Zeitdauer eines Herzzyklusses ermittelt man vorteilhaft das Intervall zwischen zwei aufeinanderfolgenden R-Wellen des EKG-Signals. Die Spitze der R-Welle lässt sich mit an sich bekannten sog. QRS-Detektionsalgorhythmen zuverlässig und mit großer Genauigkeit bestimmen. Diese Messung liefert, bezogen auf die Auflösung der Digitalisierung, ganzzahlige Werte, die als sogenanntes RR-Tachogramm gespeichert werden. Die RR-Tachogrammdaten einer Langzeitüberwachung werden in Kurzzeitblöcke segmentiert. Beispielsweise kann man aus einer 24-stündigen EKG-Überwachung 228 Blöcke von jeweils 5 Minuten Dauer gewinnen, die dann zu 288 Lorenzplots weiter verarbeitet und analysiert werden.

Die Erfindung kann insbesondere softwaretechnisch unter Zugriff auf eine Datenbank, in welcher die empirischen klinischen Daten gespeichert sind, realisiert werden. Gegenstand der vorliegenden Erfindung ist daher auch ein Computerprogramm auf einem computerlesbaren Träger, welches Programminstruktionen umfasst, um in einem Computer die Durchführung des erfindungsgemäßen Verfahrens zu bewirken. Der computerlesbare Träger kann beispielsweise ein Speichermedium, wie z.B. eine CD- oder DVD-ROM, eine Festplatte, eine Diskette oder auch ein Speicherelement eines Computers, wie ROM- oder EPROM-Speicherelemente sein. Ein computerlesbarer Träger im Sinne der vorliegenden Erfindung umfasst auch elektrische Trägersignale, wie sie beispielsweise im Internet beim Herunterladen von Programmen von einem externen Server auf einen lokalen Rechner auftreten.

Die erfindungsgemäße Vorrichtung kann beispielsweise als tragbare Vorrichtung ausgebildet und etwa in einem Notebook implementiert sein, das an sich bekannte Schnittstellen zur Dateneingabe und -ausgabe aufweist.

Gemäß einer Ausführungsform stellt die erfindungsgemäße Vorrichtung lediglich eine Auswerteeinheit dar, wobei die Eingabeeinrichtung Mittel zum Einlesen, insbesondere zum telemetrischen Empfang von ggf. digitalisierten Herzfrequenzsignalen umfasst. Sollten die Herzfrequenzsignale in analoger Form eingelesen werden, umfasst die Eingabeeinrichtung außerdem Mittel zur Digitalisierung des Herzfrequenzsignals.

Gemäß einer anderen Ausführungsform umfasst die Eingabeeinrichtung geeignete Mittel zum Erfassen und Digitalisieren von Herzfrequenzsignalen, vorzugsweise von EKG-Signalen, die beispielsweise in Form von an sich bekannten Körperelektroden-Ableitungen realisiert werden können. Die Eingabeeinrichtung kann auch Mittel zur kontinuierlichen Blutdruckerfassung oder Mittel zur Pulserfassung aufweisen, aus denen wiederum die Intervalldauern der Herzfrequenzzyklen bestimmt werden. Die Ausgabeeinrichtung der erfindungsgemäßen Vorrichtung kann Mittel zur Anzeige der ermittelten Rhythmus- bzw. Arrhythmieformen und/oder geeignete Alarmgeber zur Signalisierung von lebensbedrohlichen Arrhythmien aufweisen. Vorzugsweise sind Mittel zur telemetrischen Weiterleitung der ermittelten Rhythmus- bzw. Arrhythmieformen an eine Arztpraxis oder Klinik vorhanden. Der Datentransfer kann, beispielsweise innerhalb einer Klinik, über ein lokales Netz (LAN) oder, bei längeren Entfernungen, über Telefon oder Internet erfolgen. Bei Detektion von pathologischen Arrhythmien können ggf. zusätzlich zu der automatisch erkannten Arrhythmieform die zugehörigen Herzfrequenzsignale im Zeitbereich übertragen werden, so dass dem behandelnden Arzt weitere Informationen für die Entscheidung zur Einleitung der erforderlichen Maßnahmen zur Verfügung stehen.

Die vorliegende Erfindung weist ein breites diagnostisches Potential auf und ermöglicht beispielsweise die vollautomatisch Erkennung und Differenzierung der folgenden Rhythmus-und Arrhythmieformen:
- Sinusrhythmus mit normaler HRV
- Sinusrhythmus mit reduzierter HRV
- Sinusrhythmus mit hochgradig reduzierter HRV
- VES mit kompensatorischer Pause und fixem Kopplungsintervall
- VES mit variablem Kopplungsintervall (Parasystolie)
- VES-Bigeminus
- Polytope VES
- NSVT (nichtanhaltende ventrikuläre Tachykardie)
- VT (anhaltende ventrikuläre Tachykardien)
- SVES (supraventrikuläre Extrasystolen)
- SVES-Bigeminus
- Persistierendes AF (Vorhofflimmern)
- Paroxysmales AF (Vorhofflimmern)
- Vorhofflattern (AFL) mit alternierender AV-Überleitung
- Vorhoftachykardie (AT) mit alternierender AV-Überleitung
- Bradykardieassoziierte Pausen
- VES-assoziierte Pausen
- Artefakt-assoziierte Pausen
- Artefakte / Bandlauffehler

Die erfindungsgemäße Vorrichtung eignet sich insbesondere für Überwachungszentren, in denen eine größere Zahl von Risikopatienten in Echtzeit genau überwacht werden müssen. Hier kann die routinemäßige kontinuierliche Überwachung vollautomatisch erfolgen. Eine genauere Überwachung, ggf. unter Hinzuziehen eines Arztes, ist beispielsweise nur dann erforderlich, wenn mit dem erfindungsgemäßen Verfahren und/oder der erfindungsgemäßen Vorrichtung bestimmte pathologische Arrhythmieformen detektiert worden sind, welche die vorsorgliche oder akute Einleitung von Behandlungsmaßnahmen erforderlich machen.

Das erfindungsgemäße Verfahren bzw. die erfindungsgemäße Vorrichtung kann auch in implantierbare Defibrillatoren oder Schrittmacher integriert werden. Die so ausgerüsteten Geräte können beispielsweise die Gefahr einer lebensbedrohlichen Tachykardie durch Detektion einer Verringerung der Herzfrequenzvariabilität bereits im Vorfeld registrieren. In einem solchen Fall kann ebenfalls automatisch eine vorsorgliche Aufladung der Kondensatoren des Defibrillators veranlasst werden, so dass eine Schockabgabe unmittelbar nach Einsetzen der Tachykardie und nicht erst wie bei bekannten Defibrillatoren nach einer durch die Ladezeit der Kondensatoren bedingten Verzögerung von bis zu einer Minute erfolgen kann. Gegenstand der vorliegenden Erfindung ist daher auch ein implantierbarer Defibrillator mit einer Defibrillationsanordnung, einer Spannungsquelle und einem Impulsgeber, der einen aufladbaren Kondensator aufweist, wobei in den Defibrillator eine erfindungsgemäße Vorrichtung zur Detektion und Differenzierung von Herzrhythmusstörungen integriert ist.

Das erfindungsgemäße Verfahren und die erfindungsgemäße Vorrichtung sind extrem robust gegenüber Störeinflüssen, wie beispielsweise einer Wanderung der EKG-Grundlinie und durch Muskelartefakte hervorgerufene Störspannungen. Dies erweist sich gerade im Zusammenhang mit der Differenzierung von supraventrikulären Arrhythmien als ein großer Vorteil, da mit der vorliegenden Erfindung amplitudenschwache EKG-Wellen, wie beispielsweise P-Wellen zur Detektion und Differenzierung der Arrhythmien nicht benötigt werden.

Da gemäß einer bevorzugten Ausführungsform des erfindungsgemäßen Verfahrens lediglich eine Erkennung der QRS-Spitzen des EKGs ausreicht, eignet sich das erfindungsgemäße Verfahren insbesondere für die Arrhythmie-Überwachung unter Bedingungen des täglichen Lebens, beispielsweise auch am Arbeitsplatz und beim Sport.

Das erfindungsgemäße Verfahren führt zu einer erheblichen Steigerung der Zuverlässigkeit und Genauigkeit der Langzeit- und Kurzzeitrhythmusanalyse. Insbesondere die Kombination des erfindungsgemäßen Verfahrens mit konventioneller Langzeit-EKG- bzw. Arrhythmiebefundung führt zu einer erheblichen Verbesserung der Befundqualität.

Mit dem erfindungsgemäßen Verfahren ist auch eine erhebliche Kapazitätssteigerung von Langzeitüberwachungseinrichtungen, beispielsweise von sogenannten Holter-Labors möglich, weil einerseits weniger Personal zur Auswertung benötigt und andererseits sogar eine Ausweitung der Überwachungskapazität ermöglicht wird. Insbesondere beim kontinuierlichen Betrieb eines telemedizinischen 24-Stunden-Arrhythmieüberwachungs- und Auswertedienstes wird in Abhängigkeit von der Zahl der gleichzeitig überwachten Patienten die Zahl der pathologischen und gravierenden Arrhythmiebefunde, die ein rasches bzw. unmittelbares Handeln erfordern, ansteigen. Daher ist eine automatische Vorauswahl von normalen und pathologischen Befunden eine wesentliche Voraussetzung für eine zuverlässige und wirtschaftlich vertretbare Simultanüberwachung einer Vielzahl von Patienten.

Die Erfindung wird im folgenden anhand eines bevorzugten Ausführungsbeispiels, bei dem die Herzfrequenzsignale in Form von EKG-Signalen vorliegen, unter Bezugnahme auf die beigefügten Zeichnungen näher erläutert.

In den Zeichnungen zeigen:
- Fig. 1: den prinzipiellen Aufbau einer bevorzugten Ausführungsform der erfindungsgemäßen Vorrichtung zur automatisierten Detektion und Differenzierung von Herzrhythmusstö- rungen;
- Fig. 2: ein Ablaufschema, das die Durchführung des erfindungsgemäßen Verfahrens illust- riert;
- Fig. 3: einen schematischen Lorenzplot;
- Fig. 4: den schematischen Ablauf einer automatischen Erkennung von Mustern in einem Lorenzplot durch Clusteranalyse;
- Fig. 5: eine Veranschaulichung der Clusterfusion bei der Mustererkennung im Lorenzplot;
- Fig. 6: einen Lorenzplot mit drei detektierten Großclustern; .
- Fig. 7: eine Veranschaulichung wichtiger Parameter zur morphologischen Charakterisierung der ermittelten Großcluster
- Fig. 8: eine schematische Darstellung der automatischen Detektion von Arrhythmieformen aus Musterdaten und Zeitreihenanalysen.

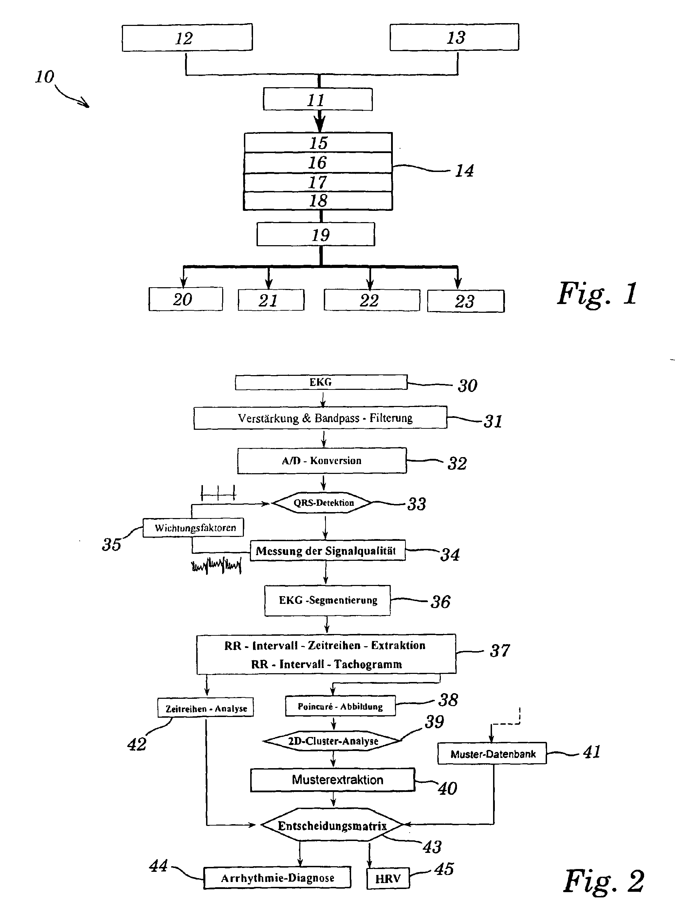

In Figur 1 ist der schematische Aufbau einer erfindungsgemäßen Vorrichtung 10 zur automatisierten Detektion und Differenzierung von Herzrhythmusstörungen dargestellt. Die erfindungsgemäße Vorrichtung umfasst eine Eingabeeinrichtung 11, welche Herzfrequenzsignale erfasst, wobei die Herzfrequenzsignale durch ein Einkanal-EKG 12 oder ein Mehrkanal-EKG 13 geliefert werden können. Die erfindungsgemäße Vorrichtung umfasst außerdem eine Verarbeitungseinrichtung 14, welche die erfassten Herzfrequenzsignale auswertet und zugeordnete Rhythmus- bzw. Arrhythmieformen ermittelt. Die Verarbeitungseinrichtung 14 kann Mittel zur EKG-Vorverarbeitung 15, beispielsweise Verstärker und/oder Filter, Analog/Digital-Konverter 16 und andere Signalaufbereitungs- und Bearbeitungsmittel aufweisen. Kernelemente der Verarbeitungseinrichtung 14 sind Mittel zur Arrhythmieanalyse 17 und Mittel zur Herzfrequenzvariabilitätsanalyse 18, die im Folgenden detaillierter erörtert werden. Die Vorrichtung 10 weist außerdem eine Ausgabeeinrichtung 19 auf, welche die ermittelten Rhythmus- bzw. Arrhythmieformen in unterschiedlicher Weise ausgeben kann, beispielsweise auf einem Monitor 20, einem Drucker 21, einem CD-Brenner 22 oder über eine Telemetrieeinrichtung 23, welche die Daten auch per Modem über Telefon- oder Datenleitungen, beispielsweise über das Internet, übertragen kann.

Die Verarbeitungseinrichtung der erfindungsgemäßen Vorrichtung kann beispielsweise in Form eines mit spezieller Analyse-Software ausgestatteten Notebooks realisiert werden. Die Eingabeeinrichtung zur Erfassung der EKG-Signale kann beispielsweise eine übliche Anordnung aus EKG-Elektroden, -Kabeln und Verteilerbox, beispielsweise für orthogonale Frank-Ableitungen, aufweisen. Die analogen EKG-Signale werden mit einer Abtastrate von 500 Hertz und einer Auflösung von 12 Bit digitalisiert und über einen Lichtwellenleiter an eine mit einem Optokoppler versehene serielle Schnittstelle des Notebooks übertragen.

Das erfindungsgemäße Verfahren zur Detektion und Differenzierung von Herzrhythmusstörungen wird nun unter Bezugnahme auf Figur 2 näher erläutert.

In einem ersten, mit der Bezugsziffer 30 bezeichneten Schritt, wird ein EKG, beispielsweise mit orthogonalen x-, y-, z-Ableitungen aufgezeichnet, anschließend im Schritt 31 verstärkt und über einen Bandpass (0,05 - 500 Hertz) gefiltert. Im Schritt 32 erfolgt die Analog/Digital-Konversion bei 12 Bit und 500 Hertz Abtastrate. Ein QRS-Detektionsalgorhythmus 33 ermittelt die Spitze der R-Welle der digitalisierten Herzzyklen. Gegebenenfalls kann für jeden Herzzyklus in einem Schritt 34 die Signalqualität ermittelt und die registrierten R-Wellen entsprechend mit Wichtungsfaktoren 35 versehen werden. Im Schritt 36 erfolgt eine Segmentierung der EKG-Zyklen in 5-Minuten-Blöcke, aus denen dann im Schritt 37 RR-Intervallzeitreihen extrahiert werden, die im dargestellten Beispiel auch als RR-Tachogramme bezeichnet werden. Die 5-Minuten-Tachogramme werden auf zwei verschiedene Weisen analysiert: Zum einen erfolgt mittels einer Poincare-Abbildung 38 eine Transformation in einen zweidimensionalen Phasenraum, der als Lorenzplot bezeichnet wird. Mittels einer zweidimensionalen Clusteranalyse 39 wird der gemessene Lorenzplot im Schritt 50 bestimmten vorgegebenen Mustern zugeordnet. Diese Muster werden mit einer empirisch erstellten Datenbank 41 verglichen, in welcher eine Korrelation der vorgegebenen Muster mit bestimmten Rhythmus- und Arrhythmieformen gespeichert ist. Zum anderen werden zur Feindiagnose bestimmte Parameter der Herzfrequenzvariabilität in einer Zeitreihenanalyse 42 aus den RR-Tachogrammen 37 ermittelt und in einer Entscheidungsmatrix 43 mit den Ergebnissen der morphologischen Auswertung verknüpft. Aufgrund dieser Daten erfolgt eine automatische Arrhythmiediagnose 44 sowie eine Bestimmung der Herzfrequenzvariabilität (HRV) 45.

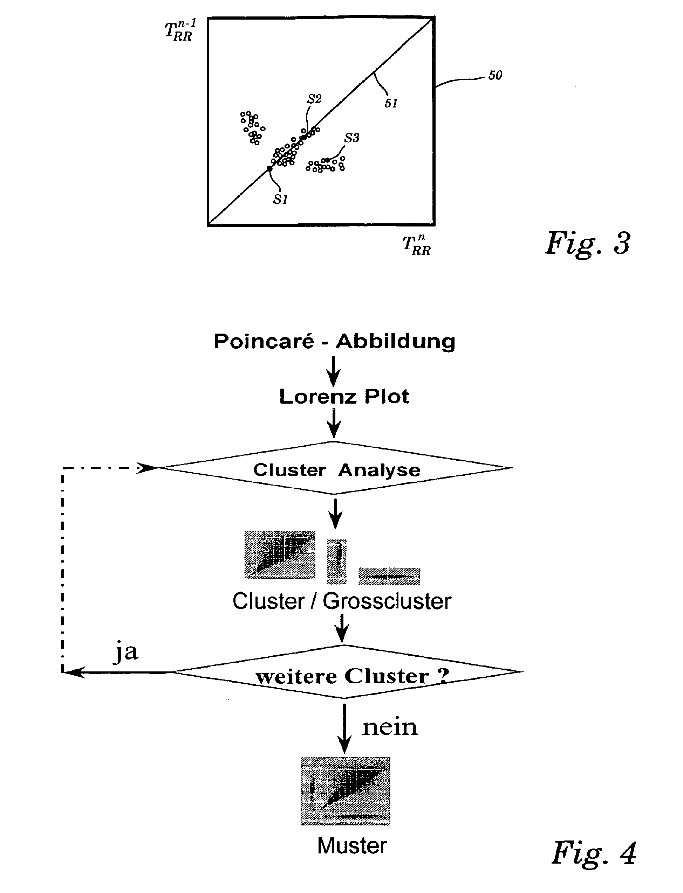

In Figur 3 ist ein aus einem RR-Tachogramm ermittelter Lorenzplot 50 schematisch dargestellt. Ein RR-Tachogramm enthält die Intervalldauern von aufeinanderfolgenden Herzzyklen, genauer gesagt die Intervalldauer zwischen aufeinanderfolgenden Spitzen der R-Welle des Herzzyklusses. Im Lorenzplot wird nun die Dauer jedes Intervalls Tⁿ_{RR} gegen die Dauer des unmittelbar vorhergehenden Intervalls Tⁿ⁻¹_{RR} abgetragen (mit Ausnahme des ersten Intervalls versteht sich, welches kein "unmittelbar vorhergehendes" Intervall besitzt). Jedem Paar von aufeinanderfolgenden Intervallen entspricht demnach ein Punkt des Lorenzplots. Die Achsenlängen des Lorenzplots wählt vorteilhaft gemäß allgemein akzeptierter Herzfrequenzkriterien von 0 bis 2000ms, so dass man einen Tachy- (0 bis 599 ms), eine Normfrequenz- (600 - 1199 ms) und eine Bradyzone (1200 - 2000 ms) unterscheiden kann. Auf der Hauptdiagonalen 51 sind die aufeinanderfolgenden Intervalle gleich lang. Man hat nun gefunden, dass eine solche Darstellung von aufeinanderfolgenden Herzzyklusdauern charakteristische Muster aus Punktwolken ergibt, die eng mit bestimmten Arrhythmieformen korrelieren. Eine solche Korrelation kann durch klinische Untersuchungen einer möglichst großen Patientenpopulation in Form einer Datenbank abgelegt werden.

Zur automatischen Analyse von Herzrhythmusstörungen ist es nun erforderlich, in einem gemessenen, d.h. aus EKG-Daten gewonnenen Lorenzplot, die vorgegebenen charakteristischen Muster automatisch wiederzuerkennen. Im hier dargestellten Ausführungsbeispiel des erfindungsgemäßen Verfahrens erfolgt die Mustererkennung mithilfe der sogenannten Clusteranalyse. Der Lorenzplot 50 wird ausgehend von drei vorgegebenen Startpositionen S1, S2 und S3 von denen vorteilhafterweise zumindest eine auf der Hauptdiagonalen 51 liegt, systematisch abgerastert. Alle in einem bestimmten Radius liegenden Punkte werden zu einem Minicluster zusammengefasst. Außerdem wird die mittlere Menge an Punktnachbarn berechnet. Unterschreitet diese einen bestimmten, als Nachbarschaftsschwelle bezeichneten Grenzwert, so wird ein Clusterrand definiert. Andernfalls wird weitergesucht, bis die Nachbarschaftsschwelle erreicht ist. Auf diese Weise entstehen größere Cluster, die allseits begrenzt sind. Im nächsten Schritt wird die Abstandsverteilung der detektierten Cluster bestimmt. Unterschreitet der Abstand zweier Cluster einen bestimmten Grenzwert D_{θ}, so werden die beiden Cluster zu einem Großcluster fusioniert. Ein geeigneter Wert D_{θ} liegt bei der Herzfrequenzanalyse im Bereich von 80 bis 120 ms, vorteilhaft bei ca. 100 ms.

In Figur 4 ist der Ablauf der Clusteranalyse schematisch zusammengefasst. Es werden zunächst einzelne Cluster ermittelt bis der gesamte Lorenzplot abgerastert ist. Die Kombination aller in einem Lorenzplot ermittelten Cluster ergibt das gesuchte Muster, welches anschließend morphologisch charakterisiert wird.

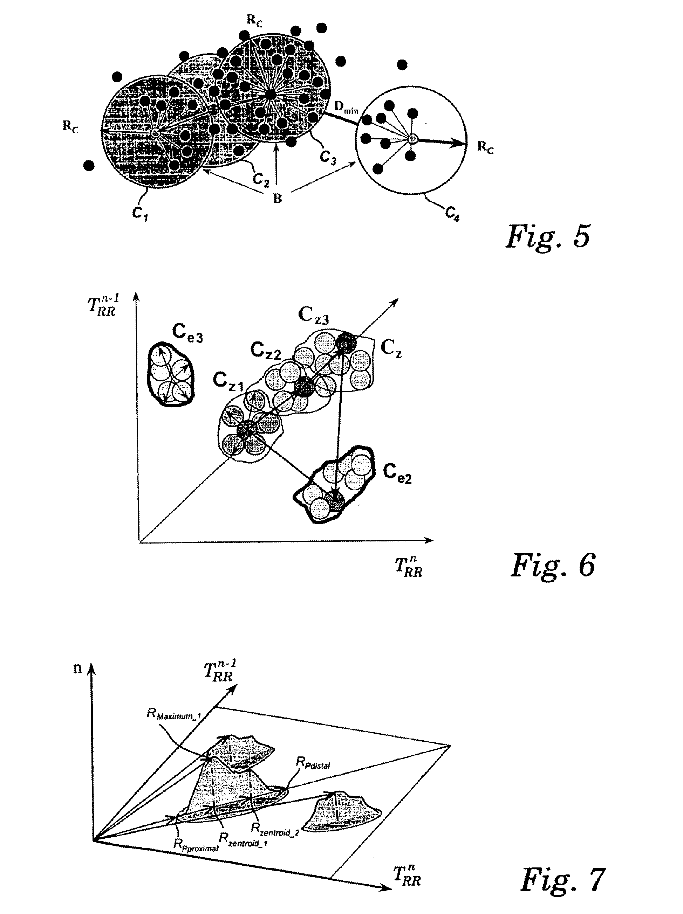

In Figur 5 ist das Vorgehen bei der Clusterfusion dargestellt. Die Cluster C1 und C3 haben einen Abstand, der geringer als der Grenzwert D_{θ} = 100 ms ist, während die Cluster C3 und C4 einen Abstand Dₘᵢₙ von mehr als 100 ms aufweisen. Demnach werden die Cluster C1 und C3 zu einem Großcluster fusioniert, wobei im Bereich C2 der Clusterrand entsprechend der Bestimmung der Nachbarschaftsschwelle angepasst wird. C4 wird nicht mit dem so gebildeten Großcluster fusioniert. Das Ergebnis einer typischen Clusteranalyse eines gesamten Lorenzplots ist in Figur 6 dargestellt. Im dargestellten Beispiel wurden ein zentraler länglicher Cluster C_{z}, der aus drei fusionierten Clustern C_{z1}, C_{z2,} und C_{z3} besteht, und zwei exzentrische Cluster Cₑ₂ und Cₑ₃ ermittelt.

Die durch die Clusteranalyse gefunden Cluster bzw. Großcluster lassen sich auf vielfältige Weise qualitativ und quantitativ charakterisieren. Im Folgenden wird ein als besonders vorteilhaft erkanntes Klassifikationsschema zur Charakterisierung des Lorenzplots unter Bezugnahme auf die schematische Darstellung der Figur 7 näher erläutert Zunächst werden, wie auch schon im Zusammenhang mit Figur 6 erwähnt, die gefundenen Cluster bezüglich ihrer Lage auf bzw. neben der Hauptdiagonalen des Lorenzplots als "zentrale" bzw. "exzentrische" Cluster charakterisiert. Außerdem werden die ermittelten Cluster geometrisch bezüglich ihrer Rand- und Mittelpunktskoordinaten parametrisiert. Wie in dem 3D-Lorenzdiagramm der Fig. 7 gezeigt, werden auch die lokalen Häufigkeitsmaxima der RR-Intervallverteilung innerhalb der Cluster bestimmt. Die jeweils dem Koordinatenursprung näher gelegen Punkte werden als "proximal" und die entfernteren Punkten als "distal" bezeichnet. In den folgenden Tabellen ist ein entsprechender Parametersatz dargestellt, der zur Charakterisierung eines Lorenzplotmusters besonders geeignet ist:

**Tabelle 1: Parameter zur Charakterisierung zentraler Cluster**

| Form-Index | | Definition |
|---|---|---|
| Distaler Randvektor | R_{Pdistal} | Vektor von 0,0 zum distalen Clusterrand |
| Proximaler Randvektor | R_{Pproximimal} | Vektor von 0,0 zum prox. Clusterrand |
| Zentroidvektor 1 | R_{zentroid_1} | Vektor von 0,0 zum proxim. Maximumfußpunkt |
| Zentroidvektor 2 | R_{zentroid_2} | Vektor von 0,0 zum distalen Maximumfußpunkt |
| Lokaler Maximumvektor 1 | R_{Maximum_1} | Vektor von 0,0 zum proximalen Maximum |
| Lokaler Maximumvektor 2 | R_{Maximum_2} | Vektor von 0,0 zum distalen Maximum |

**Tabelle 2: Parameter zur Charakterisierung proximaler exzentrischer Cluster (Proximale Maximumvektoren)**

| Form-Index | Definition |
|---|---|
| Lokaler Maximumvektor links 1 | rLM1_p |
| Lokaler Maximumvektor links 2 | rLM2_p |
| Lokaler Maximumvektor links 3 | rLM3_p |
| Lokaler Maximumvektor rechts 1 | rRM1_p |
| Lokaler Maximumvektor rechts 2 | rRM2_p |
| Lokaler Maximumvektor rechts 3 | rRM3_p |

**Tabelle 3: Parameter zur Charakterisierung distaler exzentrischer Cluster (Distale Maximumvektoren)**

| Form-Index | Definition |
|---|---|
| Lokaler Maximumvektor links 1 | rLM1_d |
| Lokaler Maximumvektor links 2 | rLM2_d |
| Lokaler Maximumvektor links 3 | rLM3_d |
| Lokaler Maximumvektor rechts 1 | rRM1_d |
| Lokaler Maximumvektor rechts 2 | rRM2_d |
| Lokaler Maximumvektor rechts 3 | rRM3_d |

Neben morphologischen Kriterien wird auch die Herzfrequenzvariabilität (HRV) mit Hilfe konventioneller Zeitbereichsindizes gemäß der nachstehenden Tabelle 4, aber auch mittels Phasenraumindizes gemäß der folgenden Tabelle 5 charakterisiert.

**Tabelle 4: HRV-Indizes aus dem Zeitbereich**

| Index | Einheit | Definition |
|---|---|---|
| RRₘᵢₙ | ms | minimales RR-Intervall |
| RRₘₐₓ, | ms | maximales RR-Intervall |
| RRₘᵢₜₜₑₗ, | ms | arithmetisches Mittel der RR-Intervallverteilung |
| RR_{modus} | ms | Modus der RR-Intervallvertellung |
| RR_{median} | ms | Median der RR-Intervallvertellung |
| SDNN | ms | Standardabweichung der mittl. RR-Intervallverteilung |
| NN50 | 1 | absolute Häufigkeit der Differenzen sukzessiver RR-Intervalle größer als 50 ms |
| rMSSD | ms | mittl. quadratische Differenz sukzessiver RR-Intervalle |
| ΔNN_{mittel,} | ms | mittlere Differenz sukzessiver RR-Intervalle |

**Tabelle 5: : HRV-Indizes aus dem Phasenbereich**

| Index | Definition |
|---|---|
| Lₘₐₓ | Maximale Länge der zentralen Punktwolke |
| Bₘₐₓ | Maximale Breite der zentralen Punktwolke |
| B₁₀₀₀ | Zentrale Punktwolkenbreite bei 1000 ms |
| B₅₀₀ | Zentrale Punktwolkenbreite bei 500 ms |

Trotz der im Detail immensen Mannigfaltigkeit der individuellen Lorenzplotmuster konnte festgestellt werden, dass bestimmte augenfällige morphologische Merkmale besonders häufig beobachtet werden können. Es wurde weiter festgestellt, dass bestimmte charakteristische Lorenzplotmuster mit bestimmten klinisch relevanten Rhythmus- und Arrhythmieformen korreliert sind.

Diese charakteristischen Muster lassen sich morphologisch, beispielsweise wie in der folgenden Tabelle 6 zusammengefasst, in übergeordnete Kategorien und diese wiederum in untergeordnete prototypische Klassen einteilen. wobei die Benennung der Klassen an dem visuellen Eindruck der jeweiligen Muster orientiert ist:

Die monomorphen Muster 1 6 bestehen aus einem einzigen zentralen Cluster, dessen Form mit einem der folgenden Attribute weiter differenziert werden konnte: konisch (= Komet), ellipsenförmig (= Torpedo), kreisförmig (= Diskus), fächerförmig (=Fächer). Die polymorphen Muster 6-10 sind aus mehreren Punktwolken zusammengesetzt. So ist der Archipeltyp durch inselförmig dislozierte zentrale und exzentrische Punktwolken gekennzeichnet. Der Z-Blatt-Typ ähnelt einer zweiblättrigen Blumenblüte, während der D-Blatt-Typ (Drei-Blatt) durch drei Nebenkeulen gekennzeichnet ist. Der Flügelpaar-Prototyp ist durch die Kombination aus einer zentralen, kometen- oder torpedoförmigen Punktwolke und zwei strikt achsensymmetrisch verlaufenden kontralateralen Nebenkeulen, die einen spitzen Winkel mit der zentralen Punktwolke bilden, gekennzeichnet. Der Drachen-Prototyp kommt in einer rautenförmigen oder in einer pfeilspitzenartigen Morphologie vor, wobei den glatten rechten und oberen Ränder der Cluster besonders augenfällig sind. Der Fragment-Prototyp ist durch eine hochsymmetrische Verteilung einer Vielzahl von Clusterfragmenten im Lorenzplot gekennzeichnet, die torpedoförmig (T), fächerförmig (F) oder D-blattförmig (D) ausgebildet sein können.

Dementsprechend lassen sich die prototypischen Muster der Kategorien Ia und Ib wiederum, wie in der Tabelle 7 dargestellt ist, durch Einführung von Subtypen weiter unterteilen. So kann, wie oben erläutert, beispielsweise der Fragment-Prototyp in einen T-, F- und D-Subtyp unterteilt werden.

**Tabelle 6: Musterkategorien und Klassen**

| Kategorie | Prototypische Klasse |
|---|---|
| | |
| Ia einfache oligomorphe Muster | 1. Komet |
| | 2. Torpedo |
| | 3. Diskus |
| | 4. Fächer |
| | |
| Ib einfache polymorphe Muster | 5. Archipel |
| | 6. Z-Blatt |
| | 7. D-Blatt |
| | 8. Flügelpaar |
| | 9. Drachen |
| | 10. Fragmente |
| | |
| II kombinierte Muster | Kombinationen zweier Musterklassen der Kategorie Ia und Ib miteinander |
| | |
| III komplexe Muster | Kombinationen aus mehr als zwei Musterklassen der Kategorien Ia und Ib |

**Tabelle 7: Subtypen der prototypischen Muster**

| **Klasse Ia** | |
|---|---|
| Komet | S-Komet (Standardmorphologie) |
| | E-Komet |
| | R-Komet |
| | B-Komet |
| Torpedo | S-Torpedo (Standardmorphologie) |
| | L-Torpedo |
| | F-Torpedo |
| Diskus | - |
| Fächer | E-Fächer |
| | D-Fächer |
| | |

| **Klasse Ib** | |
|---|---|
| Archipel | 2-Insel-Subtyp |
| | 4-Insel-Subtyp |
| | 9-Insel-Subtyp |
| Z-Blatt | Subtyp A |
| | Subtyp B |
| | Subtyp C (Lyra) |
| | Subtyp D |
| D-Blatt | Subtyp A |
| | Subtyp B (Rotor) |
| | Subtyp C (Butterfly) |
| Flügelpaar | Subtyp A |
| | Subtyp B |
| | Subtyp C |
| Fragment | T-Subtyp (torpedoförmig) |
| | F-Subtyp (fächerförmig) |
| | D-Subtyp (D-blattförmig) |

Eine detaillierte Beschreibung dieser Klassifikation und die Zuordnung der Muster zu bestimmten Rhythmus- und Arrhythmieformen auf der Grundlage einer umfangreichen klinischen Studien findet sich in der Habilitationsschrift des Anmeldens (vgl. Dr. med. Hans Dieter Esperer, Habilitationsschrift, Universität Magdeburg, 2001).

Diese vorgegebenen Muster werden nun durch empirisch ermittelte Parameter gemäß den oben beschriebenen Parametersätzen charakterisiert. Man kann die empirisch gefunden Mittelwerte dieser Parameter durch unscharfe Fuzzy-Terme beschreiben. Die Zuordnung eines gemessenen Lorenzplot-Musters zu einem vorgegeben Muster erfolgt dann nach einem als Fuzzy-Interferenz bezeichneten Schema durch ein Vielzahl von Entscheidungsregeln (WENN-DANN-Regeln), in welche auch das empirische durch klinische Studien ermittelte Expertenwissen mit eingehen kann.

Besonders bevorzugt erfolgt die Mustererkennung durch Implementierung einer Methode aus der Künstlichen Intelligenz, nämlich einer CBR - Technik (Case Based Reasoning: fallbasiertes Schließen). Die Grundlagen dieser Methode sind beispielsweise beschrieben in Lenz, M., Bartsch-Spörl, B., Burkhard, H.-D., Wess, S. (Eds.), "Case-Based Reasoning Technology - From Foundations to Applications", Lecture Notes in Artificial Intelligence 1400, Springer, 1998 oder in Aamodt, A. and Plaza, E., "Case-Based Reasoning: Foundational Issues, Methodological Variations, and System Approaches", , Al Communications, 7(1):39-59, 1994. Im Rahmen der vorliegenden Erfindung wird das zu klassifizierende Lorenzplotmuster hinsichtlich mehrerer qualitativer und quantitativer Formkriterien mit den in der Datenbasis (Lorenzplotmuster-Lexikon) gespeicherten Mustern verglichen. Das Muster, das die größte Ähnlichkeit mit dem zu analysierenden Lorenzplotmuster aufweist, wird dabei identifiziert und als Klassifizierungskategorie für das neue Muster benutzt. Die Ähnlichkeit zwischen dem zu analysierenden Muster und den in der Datenbasis vorhandenen Lorenzplotmustern wird dabei über qualitative und quantitative Ähnlichkeitsmaße bestimmt. Die qualitativen Ähnlichkeitsmaße sind im vorliegenden Fall als Ja/Nein - Entscheidungen definiert. Beispiele hierfür sind die Existenz bzw. das Fehlen eines oder mehrere Zentralcluster, die Existenz bzw. das Fehlen von paarigen bzw. unpaaren exzentrischen Clustern usw. Als quantitative Ähnlichkeitsmaße werden die euklidischen Differenzen aus geometrischen und parametrisierten Größen herangezogen. Geometrische Größen sind dabei beispielsweise Lₘₐₓ und Bₘₐₓ des/der zentralen Cluster bzw. Lₘₐₓ und Bₘₐₓ der exzentrischen Cluster (vgl. Tabelle 5). Parametrisierte Größen sind zum Beispiel charakteristische Polarkoordinaten (vgl. Tabelle 1) sowie die lokalen Häufigkeitsmaxima und -minima (vgl. Tabellen 2 und 3).

Auf der Grundlage der oben erwähnten klinischen Studien wurde unter Berücksichtigung der Daten von mehreren tausend Patienten, bei denen ein 24-Stunden-Langzeit-EKG wegen Arrhythmien oder Arrhythmieverdachts durchgeführt worden war, eine Musterdatenbank erstellt, welche die oben beschriebenen Muster und die entsprechenden klinisch ermittelten Rhythmus- und Arrhythmieformen miteinander korreliert. Als Datenbasis wurden sowohl die 24-Stunden-Daten, als auch daraus gewonnene 5-Minuten-Datensätze herangezogen.

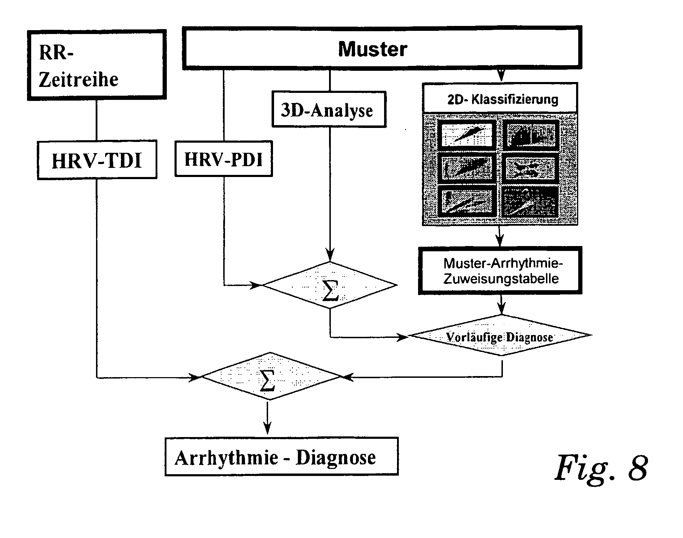

Wie in Figur 8 dargestellt werden zur endgültigen automatischen Arrhythmiediagnose nicht nur morphologische Daten der 2D- und 3D-Analyse des Lorenzplots bzw. -diagramms, sondern auch Daten der Herzfrequenzvariabilität im Phasenraum (HRV-PDI) und im Zeitbereich (HRV-TDI) herangezogen. Dementsprechend enthält auch Datenbank derartige empirisch ermittelte Kenngrößen. Schematisch ist eine Muster-Arrhythmie-Zuweisungstabelle, welche die Basis für die Zuordnung von spezifischen Arrhythmieformen zu den jeweils ermittelten Mustern bildet, in der folgenden Tabelle 8 dargestellt.

**Tabelle 8: Muster - Arrhythmie - Zuweisungsmatrix**

| **Muster** | **Arrhythmie** | |
|---|---|---|
| | **Hauptdiagnose** | **Differenzialdiagnose** |
| S - Komet | Sinusrhythmus+ normale HRV | DDD-Schrittmacher-Aktivität: atrial getriggerte Ventrikelstimulation bei DDD-Pacing mit normaler chronotroper Kompetenz |
| E-Komet | Sinusrhythmus + normale HRV | *keine* |
| R - Komet | Sinusrhythmus + normale HRV + intermittierend wandernder Vorhofschrittmacher | *keine* |
| Torpedo | Sinusrhythmus + reduzierte HRV | VVIR - Schrittmacheraktivität frequenzadaptierte Einkammerstimulation (VVIR) |
| Diskus | Sinusrhythmus + hochgradig reduzierte HRV | 1. SVT: |
| | | 1a AFL mit 1:1-ÜL |
| | | Ib AVNRT |
| | | Ic AVRT |
| | | 2. VT |
| E-Fächer | Vorhofflimmern | 1. Multifokale Tachykardie |
| | | 2. Häufige SVES |
| D-Fächer | Vorhofflimmern | *keine* |
| Archipel | Vorhofflattern mit alternierender AV- Überleitung | Vorhoftachykardie mit alternierender AV - Überleitung |
| Z-Blatt | SVES | Interponierte VES |
| D-Blatt | | |
| -Subtyp A | VES | SVES mit kompensiertem postextrasystol. Intervall |
| - Subtyp B | VES mit variabler Kopplung | *Keine* |
| - Subtyp C | VES | *Keine* |
| Flügelpaar | Bradyarrhythmieassoziierte Pausen | 1. VES-assoziierte Pausen |
| | | 2. Artefaktassoziierte Pausen |
| | | |
| F- Drachen | WI-Aktivität + Vorhofflimmern | *Keine* |
| T-Drachen | WI-Aktivität + Sinusrhythmus | *Keine* |
| Fragment | Artefakte / Bandlauffehler | *Keine* |

Das Fragment-Muster ist nur bei konventioneller Bandrekorderaufzeichnung relevant.

## Patentansprüche

1. Vorrichtung zur automatisierten Detektion und Differenzierung von Herzrhythmusstörungen mit einer Eingabeeinrichtung (11), welche Herzfrequenzsignale erfasst, einer Verarbeitungseinrichtung (14), welche die erfassten Herzfrequenzsignale auswertet und zugeordnete Rhythmus- bzw. Arrhythmieformen ermittelt, und einer Ausgabeeinrichtung (19), welche die ermittelten Rhythmus- bzw. Arrhythmieformen ausgibt, wobei die Verarbeitungseinrichtung umfasst:
Mittel zur Bestimmung der Dauer von aufeinanderfolgenden Herzschlagzyklen des Herzfrequenzsignals,
Mittel zur Korrelation jeder Zyklusdauer von zumindest einer zusammenhängenden Zeitreihe des Herzfrequenzsignals mit einer der darauffolgenden Zyklusdauern der Zeitreihe und zur Transformation der korrelierten Daten in wenigstens einen Lorenzplot, wobei die zumindest eine zusammenhängende Zeitreihe einen Zeitraum von 1 bis 15 Minuten umfasst,
Mittel zur morphologischen Charakterisierung des Lorenzplots und zur Zuordnung des Lorentzplots zu vorgegebenen Mustern, wobei die Mittel zur morphologischen Charakterisierung des Lorenzplots ausgelegt sind, eine Clusteranalyse durchzuführen und die detektierten Cluster im Phasenraum zu parametrisieren und anhand der ermittelten Parameter den vorgegebenen Mustern zuzuordnen,
Datenbankmittel, welche die vorgegebenen Muster mit bestimmten klinisch relevanten Rhythmus- bzw. Arrhythmieformen korrelieren.

2. Vorrichtung gemäß Anspruch 1, **dadurch gekennzeichnet, dass** die Eingabeeinrichtung Mittel zum telemetrischen Empfang von gegebenenfalls digitalisierten Herzfrequenzsignalen umfasst.

3. Vorrichtung gemäß Anspruch 1, **dadurch gekennzeichnet, dass** die Eingabeeinrichtung Mittel zum Erfassung und Digitalisierung von Herzfrequenzsignalen, vorzugsweise von EKG-Signalen, umfasst.

4. Vorrichtung gemäß einem der Ansprüche 1 bis 3, **dadurch gekennzeichnet, dass** diezumindest eine zusammenhängenden Zeitreihe des Herzfrequenzsignals einen Zeitraum von ,2 bis 10 min. und vorzugsweise etwa 5 min. umfasst.

5. Vorrichtung gemäß einem der Ansprüche 1 bis 4, wobei die Verarbeitungsmittel außerdem Mittel zur Bestimmung von Parametern der Herzfrequenzvariabilität mittels einer Zeitreihenanalyse umfassen.

6. Vorrichtung gemäß einem der Ansprüche 1 bis 5, **dadurch gekennzeichnet, dass** die Ausgabeeinrichtung Mittel zur telemetrischen Weiterleitung der ermittelten Rhythmus- bzw. Arrhythmieformen und gegebenenfalls der zugehörigen Herzfrequenzsignale umfasst.

7. Implantierbarer Defibrillator mit einer Defibrillationselektrodenanordnung, einer Spannungsquelle und einem Impulsgeber, der einen aufladbaren Kondensator aufweist, **dadurch gekennzeichnet, dass** der Defibrillator eine Vorrichtung nach einem der Ansprüche 1 bis 6 umfasst.

## Claims

1. A device for the automated detection and differentiation of cardiac rhythm disturbances with an input unit (11) which detects heart rate signals, a processing unit (14) which evaluates the detected heart rate signals and determines associated rhythmic or arrhythmic forms, and an output unit (19) which outputs the determined rhythmic or arrhythmic forms, said processing unit comprising:
means for determining the duration of successive heart beat cycles of the heart rate signal,
means for correlating each cycle duration of at least one coherent time series of the heart rate signal with one of the succeeding cycle durations of the time series and for transforming the correlated data into at least one Lorenz curve, the at least one coherent time series covering a time period of 1 to 15 min,
means for morphological characterization of the Lorenz curve and for assigning the Lorenz curve to predetermined patterns, wherein the means for morphological characterization of the Lorenz curve are designed for performing a cluster analysis and for parameterizing the detected clusters in the phase space and for assigning them to the predetermined patterns on the basis of the determined parameters,
databank means which correlate the predetermined patterns with certain clinically relevant rhythmic or arrhythmic forms.

2. The device as claimed in claim 1, **characterized in that** the input unit comprises means for telemetric reception of optionally digitized heart rate signals.

3. The device as claimed in claim 1, **characterized in that** the input unit comprises means for recording and digitizing heart rate signals, preferably ECG signals.

4. The device as claimed in one of claims 1 through 3, **characterized in that** the at least one coherent time series of the heart rate signal covers a time period of 2 to 10 min and preferably about 5 min.

5. The device as claimed in one of claims 1 through 4, **characterized in that** the processing means further comprise means for determining parameters of the heart rate variability by means of a time series analysis.

6. The device as claimed in one of claims 1 through 5, **characterized in that** the output unit comprises means for telemetric relay of the determined rhythmic or arrhythmic forms and, if appropriate, of the associated heart rate signals.

7. An implantable defibrillator with a defibrillation electrode arrangement, a voltage source and an impulse transmitter which has a chargeable capacitor, **characterized in that** the defibrillator comprises a device as claimed in one of claims 1 through 6.

## Revendications

1. Dispositif pour une détection et différenciation automatisées des troubles du rythme cardiaque, comprenant un dispositif d'entrée (11) captant des signaux de fréquence cardiaque, un dispositif de traitement (14) évaluant les signaux de fréquence cardiaque captés et détectant des formes de rythmes ou d'arythmies correspondantes, et un dispositif de sortie (19) qui délivre les formes de rythmes ou d'arythmies détectées, dans lequel ledit dispositif de traitement comprend :
des moyens pour déterminer la durée de cycles de battement de coeur successifs du signal de fréquence cardiaque,
des moyens pour corréler chaque durée de cycle d'au moins une série chronologique cohérente du signal de fréquence cardiaque avec l'une des durées de cycle consécutives de la série chronologique et pour transformer les données corrélées en au moins une courbe de Lorenz, dans lequel ladite au moins une série chronologique cohérente comprend une période allant de 1 à 15 minutes,
des moyens pour effectuer une caractérisation morphologique de la courbe de Lorenz et pour attribuer la courbe de Lorenz à des modèles prédéfinis, dans lequel les moyens pour la caractérisation morphologique de la courbe de Lorenz sont conçus pour effectuer une analyse de nuées et pour paramétrer les nuées détectées dans l'espace de phase et pour les attribuer aux modèles prédéfinis à l'aide des paramètres détectés,
des moyens formant base de données qui mettent en corrélation les modèles prédéfinis avec certaines formes de rythme ou d'arythmie cliniquement significatives.

2. Dispositif selon la revendication 1, **caractérisé en ce que** le dispositif d'entrée comprend des moyens pour la réception télémétrique de signaux de fréquence cardiaque éventuellement numérisés.

3. Dispositif selon la revendication 1, **caractérisé en ce que** le dispositif d'entrée comprend des moyens pour capter et numériser des signaux de fréquence cardiaque, de préférence des signaux ECG.

4. Dispositif selon l'une quelconque des revendications 1 à 3, **caractérisé en ce que** ladite au moins une série chronologique cohérente du signal de fréquence cardiaque comprend une période allant de 2 à 10 mn, et de préférence d'environ 5 mn.

5. Dispositif selon l'une quelconque des revendications 1 à 4, dans lequel les moyens de traitement comprennent en outre des moyens pour déterminer des paramètres de la variabilité de fréquence cardiaque au moyen d'une analyse de série chronologique.

6. Dispositif selon l'une quelconque des revendications 1 à 5, **caractérisé en ce que** le dispositif de sortie comprend des moyens pour le transfert télémétrique des formes de rythme ou d'arythmie détectées et le cas échéant des signaux de fréquence cardiaque associés.

7. Défibrillateur implantable avec un agencement d'électrodes de défibrillation, une source de tension et un générateur d'impulsions qui présente un condensateur rechargeable, **caractérisé en ce que** le défibrillateur comprend un dispositif selon l'une quelconque des revendications 1 à 6.
